# EUROPEAN PATENT APPLICATION

(11) **EP 4 389 676 A2**
(43) Date of publication of application: **26.06.2024**
(21) Application number: 23200663.5
(22) Date of filing: 28.09.2023
(51) Int. Cl.: B66B 31/02

(54) **SYSTEM AND METHOD FOR CLEANING AND DISINFECTING HANDRAIL, ROBOT AND PASSENGER CONVEYING EQUIPMENT**

(30) Priority: 19.12.2022 CN 202211631764
(71) Applicant: OTIS Elevator Company, Farmington, CT 06032 (US)
(72) Inventor: CHENG, Qing, Hangzhou (CN); YANG, Wenhai, Hangzhou (CN); HUANG, Qiping, Hangzhou (CN); DING, Jing, Hangzhou (CN); XU, Kaisheng, Haining City (CN)
(74) Representative: Dehns

(57) **Abstract**

The disclosure relates to a system and method for cleaning and disinfecting handrail, a robot (20) and passenger conveying equipment (10). The system for cleaning and disinfecting handrail (11) has at least one robot (20), the robot (20) comprising: a travelling device configured to enable the robot (20) to travel along a target trajectory, a cleaning and disinfecting device configured to perform cleaning and disinfection operations on a target object, and a controller connected to the travelling device and the cleaning and disinfecting device and configured to control operation of the travelling device according to command signals such that the robot (20) travels to a target position of the passenger conveying equipment (10) along the target trajectory, and to control the cleaning and disinfecting device to perform cleaning and disinfection operations on the handrail (11) at the target position. The application of the disclosure can effectively clean and disinfect the handrail (11) and improve passengers' ride experience and satisfaction, and it is beneficial to the health of the passengers.

## Description

### TECHNICAL FIELD

The present disclosure relates to the technical field of elevators, in particular to a system and method for cleaning and disinfecting handrail, a robot and passenger conveying equipment.

### BACKGROUND

Elevators have been widely used in modern society, which bring great convenience to people's daily work and life. In many places such as shopping malls, airports, subway stations, escalators with handrails are usually installed so that passengers can hold the handrails while taking the escalators. At the same time, handrails help to save passengers' physical strength and prevent accidents such as falls and injuries.

However, as escalators are frequently used by a large number of passengers, especially in public places where they often operate for about 20 hours a day, it is inevitable that a lot of dirt and bacteria and sometimes even dangerous germs, will accumulate on the surface of the handrails of the escalators due to natural environmental and passenger reasons, which is not good for human health. To this end, the cleaning method generally adopted nowadays is to arrange several cleaners to clean the escalator handrails. For example, they wipe the escalator handrails regularly with a rag and clean water to achieve the cleaning purpose.

### SUMMARY

In view of the foregoing, the present disclosure provides a system and method for cleaning and disinfecting handrail, a robot and passenger conveying equipment, so as to solve or at least alleviate one or more of the aforementioned problems and other problems in the prior art.

Firstly, according to one aspect of the present disclosure, a system for cleaning and disinfecting handrail is provided, the handrail being provided in passenger conveying equipment, wherein the system for cleaning and disinfecting handrail has at least one robot, the robot comprising:
a travelling device configured to enable the robot to travel along a target trajectory;
a cleaning and disinfecting device configured to perform cleaning and disinfection operations on a target object; and
a controller connected to the travelling device and the cleaning and disinfecting device and configured to control operation of the travelling device according to command signals so that the robot travels to a target position of the passenger conveying equipment, and to control the cleaning and disinfecting device to perform cleaning and disinfection operations on the handrail at the target position.

In a system for cleaning and disinfecting handrail according to the present disclosure, optionally, the robot further comprises a storage device, wherein the command signals are stored in the storage device and are executed regularly or irregularly by the controller; or
the system for cleaning and disinfecting handrail further comprises a control device arranged outside the robot and being in communication connection with the controller, the command signals being sent from the control device to the controller.

In a system for cleaning and disinfecting handrail according to the present disclosure, optionally, the control device comprises at least one of a master controller, a secondary controller and a mobile terminal, wherein a secondary controller is arranged in each of the passenger conveying equipment, the master controller is arranged in a system for managing respective passenger conveying equipment and is in communication connection with the secondary controller, and the mobile terminal is in communication connection with the master controller and/or the secondary controller and/or the controller, and wherein the command signals are sent to the controller from the master controller, the mobile terminal or the secondary controller in the passenger conveying equipment to be cleaned and disinfected.

In a system for cleaning and disinfecting handrail according to the present disclosure, optionally, the robot further comprises an identification device connected to the controller and configured to identify an identification feature arranged at or near the target position and associated with the passenger conveying equipment.

In a system for cleaning and disinfecting handrail according to the present disclosure, optionally, the identification device comprises one or more sensors, which include one or more of image sensors, depth sensors, video sensors and infrared sensors, and the identification feature includes an identification code.

In a system for cleaning and disinfecting handrail according to the present disclosure, optionally, the controller or the control device is configured to: instruct the robot to perform cleaning and disinfection operations on the handrail after it is determined that the identified identification feature matches the identification information of the passenger conveying equipment, and the command signals contain the identification information.

In a system for cleaning and disinfecting handrail according to the present disclosure, optionally, the robot further comprises a trigger device connected to the controller and configured to trigger a change in the state of a trigger arranged at or near the target position to identify current state of cleaning and disinfection operations when the robot starts, suspends or ends cleaning and disinfection operations after the controller or the control device determines that the identified identification feature matches the identification information.

In a system for cleaning and disinfecting handrail according to the present disclosure, optionally, the trigger includes a switch or a sensor, and the trigger is in communication with the control device.

In a system for cleaning and disinfecting handrail according to the present disclosure, optionally, the cleaning and disinfecting device comprises:
a holding part having a first portion and a second portion spaced apart from each other and defining an intermediate space therebetween, wherein the first portion and the second portion are respectively provided with a first groove and a second groove that fit two sides of the handrail, such that a space defined by the intermediate space, the first groove and the second groove is suitable for placing the handrail therein;
a container arranged inside the robot, in which a medium for cleaning and disinfection is provided; and
an operating part connected to the container and configured to perform cleaning and disinfection operations on the handrail placed in the space via an opening to the space, the cleaning and disinfection operations including spraying the medium onto the handrail.

In a system for cleaning and disinfecting handrail according to the present disclosure, optionally, the control device is configured to execute a robot mode after the robot starts cleaning and disinfection operations and to exit the robot mode after the robot ends the cleaning and disinfection operations, wherein the robot mode includes one or more of the following operations:
controlling the passenger conveying equipment to operate at a first speed, the first speed being lower than an operating speed of the passenger conveying equipment;
setting a traffic flow indicator light in the passenger conveying equipment to a preset identifier for indicating that the passenger conveying equipment is currently suspended from carrying passengers;
sending to the surrounding environment of the passenger conveying equipment a preset message for indicating that the robot is performing cleaning and disinfection operations; and
recording and storing event information that includes information relating to the cleaning and disinfection operations of the robot.

In a system for cleaning and disinfecting handrail according to the present disclosure, optionally, a wireless communication connection is built between the control device and the controller, the wireless communication modes including 5G, 4G, Wi-Fi, BlueTooth or ZigBee.

In a system for cleaning and disinfecting handrail according to the present disclosure, optionally, each of the passenger conveying equipment is provided with at least one of the robots, or at least two passenger conveying equipment share one of the robots; and/or
the passenger conveying equipment includes an escalator or a moving walkway, and the target position is an entrance or exit of the passenger conveying equipment.

Secondly, according to another aspect of the present disclosure, a method for cleaning and disinfecting handrail is also provided, the handrail being arranged in passenger conveying equipment, wherein the method for cleaning and disinfecting handrail comprises steps of:
providing at least one robot; and
driving the robot to travel to a target position of the passenger conveying equipment according to command signals, and performing cleaning and disinfection operations on the handrail at the target position.

In a method for cleaning and disinfecting handrail according to the present disclosure, optionally, the command signals are stored in the robot and executed regularly or irregularly, or the command signals are sent to the robot from a control device arranged outside the robot.

In a method for cleaning and disinfecting handrail according to the present disclosure, optionally, the control device comprises at least one of a master controller, a secondary controller and a mobile terminal, wherein a secondary controller is arranged in each of the passenger conveying equipment, the master controller is arranged in a system for managing the respective passenger conveying equipment and is in communication connection with the secondary controller, and the mobile terminal is in communication connection with the master controller and/or the secondary controller and/or the robot, and wherein the command signals are sent to the robot from the master controller, the mobile terminal or the secondary controller in the passenger conveying equipment to be cleaned and disinfected; and/or
a wireless communication connection is built between the control device and the robot, the wireless communication modes including 5G, 4G, Wi-Fi, BlueTooth or ZigBee.

In a method for cleaning and disinfecting handrail according to the present disclosure, optionally, the method further comprises steps of: causing the robot to identify an identification feature arranged at or near the target position, and instructing the robot to perform cleaning and disinfection operations on the handrail after it is determined that the identified identification feature matches identification information of the passenger conveying equipment, wherein the identification feature is associated with the passenger conveying equipment, the identification feature includes an identification code, and the command signals contain the identification information.

In a method for cleaning and disinfecting handrail according to the present disclosure, optionally, the method further comprises step of: causing the robot to trigger a change in the state of a trigger arranged at or near the target position to identify current state of cleaning and disinfection operations when the robot starts, suspends or ends cleaning and disinfection operations after it is determined that the identified identification feature matches the identification information.

In a method for cleaning and disinfecting handrail according to the present disclosure, optionally, a robot mode is executed after the robot starts cleaning and disinfection operations and the robot mode is exited after the robot ends the cleaning and disinfection operations, the robot mode including one or more of the following operations:
controlling the passenger conveying equipment to operate at a first speed, the first speed being lower than an operating speed of the passenger conveying equipment;
setting a traffic flow indicator light in the passenger conveying equipment to a preset identifier for indicating that the passenger conveying equipment is currently suspended from carrying passengers;
sending to the surrounding environment of the passenger conveying equipment a preset message for indicating that the robot is performing cleaning and disinfection operations; and
recording and storing event information including information relating to the cleaning and disinfection operations of the robot.

In a method for cleaning and disinfecting handrail according to the present disclosure, optionally, each of the passenger conveying equipment is provided with at least one of the robots, or at least two passenger conveying equipment share one of the robots; and/or
the passenger conveying equipment includes an escalator or a moving walkway, and the target position is an entrance or exit of the passenger conveying equipment.

In addition, according to yet another aspect of the present disclosure, passenger conveying equipment is also provided, wherein the passenger conveying equipment is configured with the system for cleaning and disinfecting handrail according to any of the above.

Furthermore, according to still another aspect of the present disclosure, a robot for passenger conveying equipment is further provided, which comprises:
a travelling device configured to enable the robot to travel along a target trajectory;
a cleaning and disinfecting device configured to perform cleaning and disinfection operations on a target object; and
a controller connected to the travelling device and the cleaning and disinfecting device and configured to control operation of the travelling device according to command signals such that the robot travels to a target position of the passenger conveying equipment, and to control the cleaning and disinfecting device to perform cleaning and disinfection operations on the handrail at the target position.

In a robot according to the present disclosure, optionally, the robot further comprises a storage device, wherein the command signals are stored in the storage device and are executed regularly or irregularly by the controller; or
the controller is further in communication connection with a control device arranged outside the robot, wherein the control device is associated with the passenger conveying equipment and sends the command signals to the controller.

In a robot according to the present disclosure, optionally, the robot further comprises an identification device connected to the controller and configured to identify an identification feature arranged at or near the target position, wherein the identification feature is associated with the passenger conveying equipment and include identification codes.

In a robot according to the present disclosure, optionally, the controller or the control device is configured to: instruct the robot to perform cleaning and disinfection operations on the handrail after it is determined that the identified identification feature matches identification information of the passenger conveying equipment, where the command signals contain the identification information.

In a robot according to the present disclosure, optionally, the robot further comprises a trigger device connected to the controller and configured to: trigger a change in the state of a trigger arranged at or near the target position to identify current state of cleaning and disinfection operations when the robot starts, suspends or ends the cleaning and disinfection operations after the controller or the control device determines that the identified identification feature matches the identification information.

In a robot according to the present disclosure, optionally, the cleaning and disinfecting device comprises:
a holding part having a first portion and a second portion and an intermediate space defined thereby, wherein the first portion and the second portion are respectively provided with a first groove and a second groove that fit two sides of the handrail such that a space defined by the intermediate space, the first groove and the second groove is suitable for placing the handrail therein;
a container arranged inside the robot, in which a medium for cleaning and disinfection is provided; and
an operating part connected to the container and configured to perform cleaning and disinfection operations on the handrail placed in the space, the cleaning and disinfection operations including spraying the medium onto the handrail.

By adopting the technical solutions of the present disclosure, the handrail of the passenger conveying equipment can be effectively cleaned and disinfected by exploiting the robots, which promotes the service level of the passenger conveying equipment, improves passengers' ride experience and satisfaction, and is beneficial to the health of the passengers. In addition, the utilization of robots can also significantly improve work efficiency while reducing overall costs.

### BRIEF DESCRIPTION OF THE DRAWINGS

The technical solutions of the present disclosure will be described in further detail below with reference to the accompanying drawings and embodiments. However, it should be understood that these drawings are designed merely for the purpose of explanation and only intended to conceptually illustrate the structures and configurations described herein, and are not required to be drawn to scale.
FIG 1 is a schematic diagram showing an application scenario of an embodiment of a robot according to the present disclosure being used for cleaning and disinfecting the handrail of passenger conveying equipment.
FIG 2 is a three-dimensional structural schematic diagram of the embodiment of the robot shown in FIG 1.
FIG 3 is a process flow diagram of an embodiment of a method for cleaning and disinfecting handrail according to the present disclosure.

### DETAILED DESCRIPTION

Firstly, it should be noted that the structure, composition, steps, characteristics, advantages and the like of the system and method for cleaning and disinfecting handrail, the robot and the passenger conveying equipment according to the present disclosure will be described below by way of examples. However, neither of the descriptions should be understood as limiting the present disclosure in any way. In the text, the technical terms "first", "second" and "third" are only used for the purpose of distinguishing and are not intended to indicate the order and relative importance thereof. The technical term "robot" refers generally to all man-made machines capable of performing tasks automatically according to instructions.

In addition, for any single technical feature described or implied in the embodiments mentioned herein, or any single technical feature shown or implied in individual drawings, the present disclosure still allows for any combination or deletion of these technical features (or equivalents thereof) without any technical obstacle. Therefore, it should be considered that further embodiments according to the present disclosure are also within the scope of the present disclosure.

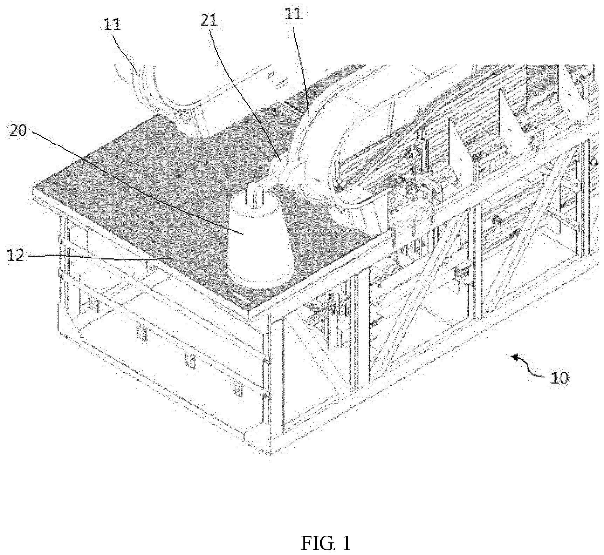

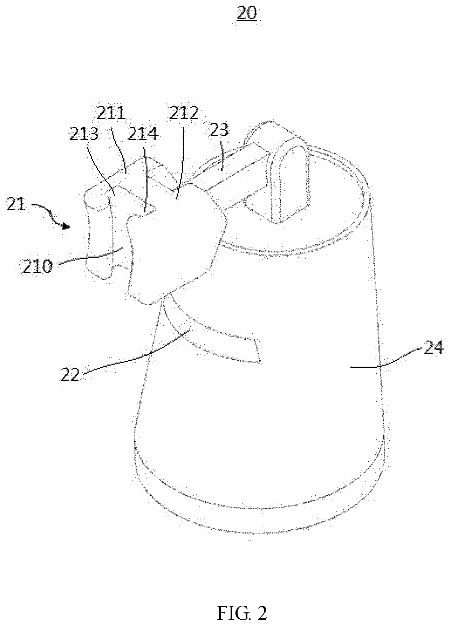

Referring to FIG 1, a local structure of an example of passenger conveying equipment is shown. It also demonstrates an application scenario of cleaning and disinfecting the handrail of the passenger conveying equipment using an embodiment of the robot according to the present disclosure. FIG 2 further illustrates a general structure of the embodiment of the robot. The solution of the present disclosure will be described in detail below through the above example.

As shown in FIGS. 1 and 2, a robot 20 is provided for cleaning and disinfecting the handrail 11 of the passenger conveying equipment 10. The robot 20 may be configured with a travelling device, a cleaning and disinfecting device and a controller.

Specifically, the travelling device is connected to the controller in any feasible ways to achieve the motion function of the robot 20, i.e., to enable the robot to travel to a desired position, such as moving to the destination along a target trajectory as instructed by the controller (e.g., moving forward, backward or up or down by means of the conveyor belt or steps of the passenger conveying equipment). Depending on the application requirements, a travelling device may be in the form of a step driving mechanism, a peristaltic driving mechanism, a serpentine driving mechanism, or a hybrid driving mechanism, and may be configured with components such as rollers, gear transmission mechanisms, hydraulic mechanisms, position detection elements, sensors, cables, and the like. Many implementations for robot walking have been provided in the prior art, so the present disclosure does not impose any restrictions in the regard.

In the robot 20, a cleaning and disinfecting device is used to clean and disinfect the handrail. It should be appreciated that "cleaning and disinfection" in the text includes the removal of dirt, bacteria, germs, etc. from the target object using any method or methods, such as cleaning, disinfection and/or sterilization, and allows the use of possible cleaning solutions, disinfectants, ultraviolet radiation, dryers, rags, and the like.

A portion of the structure of a cleaning and disinfecting device that may be configured in the robot 20 is shown in FIG 2. As shown in the figure, one or more containers may be placed in the internal space 24 of the robot 20, where one or more mediums for cleaning and disinfection (e.g., water, alcohol and/or other disinfectants, etc.) may be stored therein. The mediums can then be sprayed onto the handrail 11 through an operating part (e.g., a spray head, etc.) connected to the container(s) when necessary to achieve the cleaning and disinfection effect.

A holding part 21 for holding the handrail in the cleaning and disinfecting device is shown in FIG 2. It has a first portion 211 and a second portion 212, where the two parts are partially separated from each other to form an intermediate space for accommodating the handrail. A groove 213 is provided on the first portion 211, and a groove 214 is provided on the second portion 212, where the two grooves fit the two sides of the handrail 11 respectively. A space 210 is thus defined by the intermediate space formed by the spacing between the first portion 211 and the second portion 212, together with the groove 213 and the groove 214, which is more suitable for stably accommodating the handrail 11 therein. The handrail 11 is then cleaned and disinfected when it passes through this space along the handrail rail. For example, on the one hand, the first portion 211 and the second portion 212 may be connected using a support part 23 with a hollow cavity at this point, and on the other hand, the operating part (e.g., spray head, wiping rag, ultraviolet lamp) may be optionally arranged to pass through the cavity to perform cleaning and disinfection operations on the running handrail 11 via the opening to space 210. FIG 1 has schematically shown the scenario that the handrail 11 has been clamped in the holding part 21 for cleaning and disinfection.

As an example, at least one part of each of the first portion 211 and the second portion 212 may be made of elastic material such as rubber in order to better contact the handrail 11 and avoid or reduce collisions, abrasions and the like between them. In addition, as an alternative, it is advantageous to construct the two parts 211, 222 and their corresponding grooves 213, 214 into symmetrical structures. Furthermore, considering that there may be different application requirements, it is allowable by the solutions of the present disclosure that the operating part may perform cleaning and disinfection operations on the handrail located in the space 210 and/or outside the space and in adjacent locations from a single direction or from multiple directions at the same time (such as from above, below or other possible directions).

In the robot 20, the controller is mainly used to achieve control functions. It is connected to the aforementioned travelling device and cleaning and disinfecting device, and controls them. Specifically, the controller may be configured to control the operation of the travelling device according to command signals, so that the robot 20 can travel to a target position of the passenger conveying equipment 10, where the handrail 11 can be cleaned and disinfected using a cleaning and disinfecting device. The target position may be selected and set according to the application requirements, such as the entrance and/or exit of the passenger conveying equipment 10, and the like. It is schematically denoted with reference numeral 12 in FIG 1.

With respect to the command signal, it can take any feasible form of data which may include any relevant content related to the task of cleaning and disinfecting the handrail at the target location of the passenger conveying equipment, such as the feature information (e.g., machinery equipment number), route, navigation information, start time and duration of the cleaning and disinfection task, interaction information with the passenger conveying equipment or other machinery equipment or systems, etc. of the passenger conveying equipment to be cleaned and disinfected.

It is noteworthy that, in one or more embodiments, command signals may be stored directly in the robot. For example, storage devices (e.g., storage chips, storage cards, USB drives, etc.) may be optionally configured in the robot to store such command signals locally on the robot, so that command signals may be called from the storage devices by the controller regularly or irregularly. For example, the robot may be placed in suitable locations at ordinary times, such as lobby corners, special areas or warehouses where the passenger conveying equipment is installed. And then, based on, for example, fixed time points (e.g., the generally idle period of the passenger conveying equipment, such as 24:00, 2:30, 5:00, etc.) or time intervals (e.g., every 60 minutes, 90 minutes, etc.) per day, or based on fixed time points per week (e.g., 24:00 on Monday, 4:30 on Wednesday, etc.) and other preset operating time points, the controller will automatically call the command signals stored locally to perform the cleaning and disinfection task for the handrail at the target location of the passenger conveying equipment, which is therefore very convenient and efficient.

In addition, in one or more embodiments, command signals may also be sent to the controller in the robot by means of a control device arranged outside the robot, thus enabling the controller to start controlling the travelling device and the cleaning and disinfecting device to start working upon receipt of the command signals. Communication connections between the aforementioned control device and the controller in the robot may be wired, wireless, or combinations thereof. When wireless communication is used, it may include, but is not limited to, one or more of, for example, 5G, 4G, Wi-Fi, BlueTooth, ZigBee. That is, the present disclosure allows for appropriate selections and adjustments according to the specific requirements of various applications.

With respect to the control device, it may be implemented using any suitable component, chip, module, device or equipment. For example, one or more of a master controller, a secondary controller and a mobile terminal may be optionally configured to act as a control device. Wherein, the master controller is a controller arranged in a system used to manage the respective passenger conveying equipment. Whereas, each of passenger conveying equipment is usually provided with a secondary controller for operation management and control. These secondary controllers may be in communication connection with the master controller, so that the master controller can manage and control the operation of each of the passenger conveying equipment. The mobile terminal (e.g., mobile phone, dedicated terminal, etc.) may be used to communicate and interact with the master controller, secondary controller and/or controller in the robot at anytime and anywhere by installing application programs, which is convenient, fast and efficient. As an example, the control device can optionally be configured to include the master controller, the secondary controller and the mobile terminal at the same time, thus causing more ways to communicate with the controller in the robot so that one of these ways may be flexibly selected to send a command signal to the robot as the circumstances require.

In some application scenarios, the control device may be configured to determine whether to execute or exit the robot mode depending on the operating condition of the robot, i.e. the above mode may be executed once it is determined that the robot has started the cleaning and disinfection operations, and the robot mode may be exited once the cleaning and disinfection operations are completed. Optionally, the robotic mode may be configured to include one or more of the following operations:
controlling the passenger conveying equipment to operate at a specific speed, the specific speed being lower than the operating speed of the passenger conveying equipment 10, so as to facilitate the cleaning and disinfection of the handrail moving at this speed, where normally the above speed can directly be the maintenance speed of the passenger conveying equipment;
adjusting and configuring a traffic flow indicator lamp or similar device in the passenger conveying equipment to a preset indicator, where such a preset indicator is used to indicate that the passenger conveying equipment is currently in the state of being suspended from carrying passengers, e.g., displayed as "-" and the like, so as to promptly remind and inform the passengers so as to avoid accidents;
sending a preset message to the surrounding environment of the passenger conveying equipment, where such a preset message is used to indicate that the equipment is currently being cleaned and disinfected by a robot, for example, using a voice broadcast to remind that "the robot is performing cleaning and disinfection operations, so please pay attention to safety", or displaying the text "robot is operating" on an electronic screen that may be equipped to alert the passengers, so as to enhance system safety;
recording and storing the event information involved, where such event information usually includes information related to the cleaning and disinfection operations of the robot, such as the start time, end time, etc., which is helpful for subsequent query, analysis and statistics.

Depending on the actual application requirements, more possible configuration designs or modifications may be made to the robot according to the present disclosure. For example, an identification device 22 may be provided on the robot as shown in FIG 1, e.g., using one or more sensors (e.g., image sensor, depth sensor, video sensor, and/or infrared sensor, etc.), so as to identify an identification feature placed at or near the target position of the passenger conveying equipment. The identified identification feature is then sent to the controller located in the robot or to the external control device as mentioned above. And, the robot is instructed not to perform the cleaning and disinfection operations on the handrail 11 unless it is determined by the controller or the control device that the identified identification feature fully matches the identification information of the passenger conveying device 10. By adding the above validation process, it can prevent possible misoperations by the robot on the current passenger conveying equipment which does not need cleaning and disinfection, thus effectively improving the accuracy and reliability of system operation and avoiding undesired waste and loss in time, resources and equipment.

With respect to the aforementioned identification information, it is associated with the passenger conveying equipment and may be in the form of various types of data information to indicate the uniqueness of each of passenger conveying equipment, such as a QR (Quick Response) code, a DM (Data Matrix) code and other identification codes. Such an identification information may be positioned in a suitable place by pasting, screwing and other appropriate means, such as on the wall near the entrance/exit of the passenger conveying equipment. With respect to the above identification feature, it may have characteristics similar to the identification information, i.e., it is the unique identity characteristics used to identify the passenger conveying equipment to be cleaned and disinfected, it can take the form of data identical to or associated with the identification information, and it may be coded into the command signals as mentioned above for the robot to obtain.

For another example, a trigger device may be provided on the robot, and a corresponding trigger (e.g., a switch or sensor) may be placed at or near the target position of the passenger conveying device at the same time. Thus, when the controller in the robot or the external control device determines that the identification feature identified by the identification device match the identification information, the trigger device of the robot may be used to trigger a change in the state of a trigger accordingly (e.g., by touching a switch component to place it in a different first, second or third position, or by turning on, flashing or turning off a sensor display lamp) to clearly indicate the current state of cleaning and disinfection operation, when the robot starts, suspends or ends cleaning and disinfection operations. This is very helpful for timely display and determination of the current state, so that the system can operate safely or take corresponding measures. For example, in the case when the trigger is in communication connection with the control device, the control device can adjust the system operation according to the above state changes, such as executing or exiting the aforementioned robotic mode, and the like.

It should be noted that, as shown in FIG 1, only one robot is configured for cleaning and disinfecting passenger conveying equipment. However, it should be appreciated that the present disclosure allows two, three or more robots to be configured for a piece of passenger conveying equipment. These robots can perform cleaning and disinfection operations in parallel on the handrail at different positions of the passenger conveying equipment, thereby improving work efficiency. In addition, the present disclosure also allows two or more pieces of passenger conveying equipment to share a single robot for cleaning and disinfection operations, while their respective cleaning and disinfection operations may be staggered from each other as required, which will help to save costs and avoid arranging too many robots to occupy to much space.

With regard to the robot, its specific model, size, working power, service area, working time, cleaning and disinfection contents (e.g., dirt removal, disinfection or sterilization using chemical agents such as alcohol, disinfectants, etc., disinfection or sterilization using physical devices such as ultraviolet lamp, ozone generator), etc. can be flexibly selected, set or adjusted according to the application requirements. The robot may be configured with batteries as a power source to power its components, or may be connected directly to the power grid or other power sources to obtain electric power using cables. The latter solution is feasible when the robot is arranged relatively close to the handrail of the passenger conveying equipment to be cleaned and disinfected.

The above details the basic configuration, working conditions, etc. of the robot according to the present disclosure. A system for cleaning and disinfecting handrail according to the present disclosure may be formed by configuring one or more such robots and applying them to passenger conveying equipment. Or, a system for cleaning and disinfecting handrail according to the present disclosure can also be formed by combining such robots with the external control device as discussed above. In this way, the system for cleaning and disinfecting handrail can advantageously replace the widely used manual cleaning method and realize the automatic and intelligent cleaning and disinfection of the passenger conveying equipment. It not only ensures a healthy environment and improves passengers' ride experience, but also helps to reduce the overall cost. It is particularly suitable for automatically starting the cleaning and disinfection operations on the equipment in the non-operational hours in the late night, thus solving the longstanding problem that passengers will be affected by manual work during the day as cleaners have to work at daytime.

According to the solution of the present disclosure, passenger conveying equipment is also provided. That is, the passenger conveying equipment may be equipped with the aforementioned system of cleaning and disinfecting handrail, so as to achieve outstanding technical effects significantly better than the prior art. It should be appreciated that the passenger conveying equipment may include, but not be limited to, escalators, moving walkways, etc., which may be installed and arranged in an indoor or outdoor environment as required by the application.

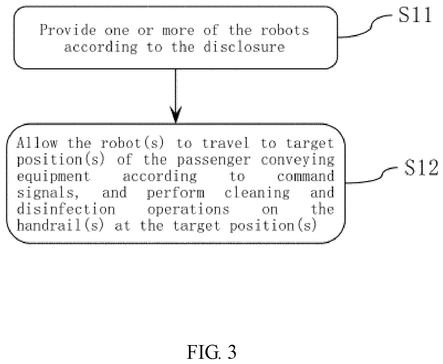

With continued reference to FIG 3, an embodiment of a method of cleaning and disinfecting handrail according to the present disclosure is shown. In this embodiment, the following steps may be included:
in step S11, one or more robots may be provided, e.g., the aforementioned robot(s) according to the disclosure may be arranged at suitable places as required for cleaning and disinfecting the handrail(s) at the target position(s) of the passenger conveying equipment;
   and
in step 12, the robot(s) may be made, according to command signals, to perform cleaning and disinfection operations on the handrail(s) at the aforementioned target position(s).

It would be appreciated by those skilled in the field that, as the technical contents such as robot and its components, control device, robotic mode, passenger conveying equipment, etc. have been described in great detail in the foregoing, e.g., the robotic mode may be executed (or exited) after the robot starts (or ends) the cleaning and disinfection operations, the operation steps of identifying the identification feature may be performed by the robot prior to the cleaning and disinfection operations, and triggering operation steps may be performed by the robot to indicate the current state of the cleaning and disinfection operations. Therefore, by directly referring to the specific descriptions and contents of the corresponding parts as mentioned above, further possible steps for the method of cleaning and disinfecting handrail may be formed, which will not be repeated here.

The system and method for cleaning and disinfecting handrail, the robot and the passenger conveying equipment according to the present disclosure have been described above in detail by way of example only. These examples are merely used to illustrate the principles and embodiments of the disclosure, rather than limiting the present disclosure. Various modifications and improvements can be made by those skilled in the art without departing from the scope of the disclosure. Therefore, all equivalent technical solutions should fall within the scope of the disclosure and be defined by the claims of the disclosure.

## Claims

1. A robot for passenger conveying equipment, comprising:
a travelling device configured to enable the robot to travel along a target trajectory;
a cleaning and disinfecting device configured to perform cleaning and disinfection operations on a target object; and
a controller connected to the travelling device and the cleaning and disinfecting device and configured to control operation of the travelling device according to command signals such that the robot travels to a target position of the passenger conveying equipment, and to control the cleaning and disinfecting device to perform cleaning and disinfection operations on the handrail at the target position.

2. The robot according to claim 1, wherein the robot further comprises a storage device, wherein the command signals are stored in the storage device and are executed regularly or irregularly by the controller; or
the controller is further in communication connection with a control device arranged outside the robot, wherein the control device is associated with the passenger conveying equipment and sends the command signals to the controller.

3. The robot according to claim 1 or 2, wherein the robot further comprises an identification device connected to the controller and configured to identify an identification feature arranged at or near the target position, wherein the identification feature is associated with the passenger conveying equipment and include identification codes.

4. The robot according to claim 3, wherein the controller or the control device is configured to: instruct the robot to perform cleaning and disinfection operations on the handrail after it is determined that the identified identification feature matches identification information of the passenger conveying equipment, where the command signals contain the identification information.

5. The robot according to claim 4, wherein the robot further comprises a trigger device connected to the controller and configured to: trigger a change in the state of a trigger arranged at or near the target position to identify current state of cleaning and disinfection operations when the robot starts, suspends or ends the cleaning and disinfection operations after the controller or the control device determines that the identified identification feature matches the identification information.

6. The robot according to any preceding claim, wherein the cleaning and disinfecting device comprises:
a holding part having a first portion and a second portion and an intermediate space defined thereby, wherein the first portion and the second portion are respectively provided with a first groove and a second groove that fit two sides of the handrail such that a space defined by the intermediate space, the first groove and the second groove is suitable for placing the handrail therein;
a container arranged inside the robot, in which a medium for cleaning and disinfection is provided; and
an operating part connected to the container and configured to perform cleaning and disinfection operations on the handrail placed in the space, the cleaning and disinfection operations including spraying the medium onto the handrail.

7. A system for cleaning and disinfecting handrail, the handrail being arranged in passenger conveying equipment, wherein the system for cleaning and disinfecting handrail has at least one robot according to any of claims 1 to 6.

8. The system for cleaning and disinfecting handrail according to claim 7;
wherein the robot further comprises a storage device, wherein the command signals are stored in the storage device and are executed regularly or irregularly by the controller; or
the system for cleaning and disinfecting handrail further comprises a control device arranged outside the robot and being in communication connection with the controller, the command signals being sent from the control device to the controller; and/or
wherein each of the passenger conveying equipment is provided with at least one of the robots, or at least two passenger conveying equipment share one of the robots; and/or
wherein the passenger conveying equipment includes an escalator or a moving walkway, and the target position is an entrance or exit of the passenger conveying equipment; and/or
wherein the robot further comprises an identification device connected to the controller and configured to identify an identification feature arranged at or near the target position and associated with the passenger conveying equipment,
optionally wherein the identification device comprises one or more sensors, which include one or more of image sensors, depth sensors, video sensors and infrared sensors, and the identification feature includes an identification code.

9. The system for cleaning and disinfecting handrail according to claim 7, when dependent on claim 2;
wherein the control device comprises at least one of a master controller, a secondary controller and a mobile terminal, wherein a secondary controller is arranged in each of the passenger conveying equipment, the master controller is arranged in a system for managing respective passenger conveying equipment and is in communication connection with the secondary controller, and the mobile terminal is in communication connection with the master controller and/or the secondary controller and/or the controller, and wherein the command signals are sent to the controller from the master controller, the mobile terminal or the secondary controller in the passenger conveying equipment to be cleaned and disinfected; and/or
wherein the control device is configured to execute a robot mode after the robot starts cleaning and disinfection operations and to exit the robot mode after the robot ends the cleaning and disinfection operations, the robot mode comprising one or more of the following operations:
controlling the passenger conveying equipment to operate at a first speed, the first speed being lower than an operating speed of the passenger conveying equipment;
setting a traffic flow indicator light in the passenger conveying equipment to a preset identifier for indicating that the passenger conveying equipment is currently suspended from carrying passengers;
sending to the surrounding environment of the passenger conveying equipment a preset message for indicating that the robot is performing cleaning and disinfection operations; and
recording and storing event information that includes information relating to the cleaning and disinfection operations of the robot; and/or
wherein a wireless communication connection is built between the control device and the controller, the wireless communication modes including 5G, 4G, Wi-Fi, BlueTooth or ZigBee.

10. The system for cleaning and disinfecting handrail according to claim 7, when dependent on claim 5, wherein the trigger includes a switch or a sensor, and the trigger is in communication with the control device.

11. A method for cleaning and disinfecting handrail, the handrail being arranged in passenger conveying equipment, wherein the method comprises steps of:
providing at least one robot; and
driving the robot to travel to a target position of the passenger conveying equipment according to command signals, and performing cleaning and disinfection operations on the handrail at the target position.

12. The method for cleaning and disinfecting handrail according to claim 11; wherein the command signals are stored in the robot and executed regularly or irregularly, or the command signals are sent to the robot from a control device arranged outside the robot,
optionally wherein the control device comprises at least one of a master controller, a secondary controller and a mobile terminal, wherein a secondary controller is arranged in each of the passenger conveying equipment, the master controller is arranged in a system for managing the respective passenger conveying equipment and is in communication connection with the secondary controller, and the mobile terminal is in communication connection with the master controller and/or the secondary controller and/or the robot, and wherein the command signals are sent to the robot from the master controller, the mobile terminal or the secondary controller in the passenger conveying equipment to be cleaned and disinfected; and/or
a wireless communication connection is built between the control device and the robot, the wireless communication modes including 5G, 4G, Wi-Fi, BlueTooth or ZigBee.

13. The method for cleaning and disinfecting handrail according to claim 11 or 12 further comprising steps of:
causing the robot to identify an identification feature arranged at or near the target position, and instructing the robot to perform cleaning and disinfection operations on the handrail after it is determined that the identified identification feature matches identification information of the passenger conveying equipment, wherein the identification feature is associated with the passenger conveying equipment, the identification feature includes an identification code, and the command signals contain the identification information,
optionally further comprising the step of: causing the robot to trigger a change in the state of a trigger arranged at or near the target position to identify current state of cleaning and disinfection operations when the robot starts, suspends or ends cleaning and disinfection operations after it is determined that the identified identification feature matches the identification information.

14. The method for cleaning and disinfecting handrail according to claim 11, 12 or 13; wherein a robot mode is executed after the robot starts cleaning and disinfection operations and the robot mode is exited after the robot ends the cleaning and disinfection operations, the robot mode including one or more of the following operations:
controlling the passenger conveying equipment to operate at a first speed, the first speed being lower than an operating speed of the passenger conveying equipment;
setting a traffic flow indicator light in the passenger conveying equipment to a preset identifier for indicating that the passenger conveying equipment is currently suspended from carrying passengers;
sending to the surrounding environment of the passenger conveying equipment a preset message for indicating that the robot is performing cleaning and disinfection operations; and
recording and storing event information including information relating to the cleaning and disinfection operations of the robot; and/or
wherein each of the passenger conveying equipment is provided with at least one of the robots, or at least two passenger conveying equipment share one of the robots; and/or
wherein the passenger conveying equipment includes an escalator or a moving walkway, and the target position is an entrance or exit of the passenger conveying equipment.

15. A passenger conveying equipment, wherein the passenger conveying equipment is configured with the system for cleaning and disinfecting handrail according to any of claims 7-9.
